# EUROPEAN PATENT APPLICATION

(11) **EP 3 569 718 A1**
(43) Date of publication of application: **20.11.2019**
(21) Application number: 18382334.3
(22) Date of filing: 16.05.2018
(51) Int. Cl.: C12Q 1/6883

(54) **KIT AND METHOD FOR DETERMINING THE RECEPTIVITY STATUS OF AN ENDOMETRIUM**

(71) Applicant: Integrated Genetic Lab Services SLU, 03540 Alicante (ES)
(72) Inventor: AIZPURUA SÁENZ, Jon, 03540 Alicante (ES); SARASA MARCUELLO, Jonás, 03540 Alicante (ES); ENCISO LORENCES, María, 03540 Alicante (ES)
(74) Representative: Elzaburu S.L.P.

(57) **Abstract**

The present invention relates to a kit for determining the receptivity status of an endometrium comprising means for quantifying the expression of a set of genes comprising the genes AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1 as well as to a method for determining the receptivity status of an endometrium comprising the quantification of the expression of said gene set or said individual genes.

## Description

### FIELD OF THE INVENTION

The present invention belongs to the field of reproductive medicine. The present invention relates to a kit for determining the receptivity status of an endometrium comprising means for quantifying the expression of several genes as well as to a method for determining the receptivity status of an endometrium comprising the quantification of the expression of said genes.

### BACKGROUND OF THE INVENTION

One of the key processes for the establishment of a successful pregnancy is embryonic implantation into the endometrium. Implantation is a complex process that involves an intricate dialog between the embryo and the endometrial cells (Singh et al., 2011). This interaction is essential for the apposition, adhesion and invasion of the blastocyst in the human endometrium (Giudice and Irwin, 1999).

The human endometrium is a highly dynamic structure, which undergoes periodical changes during the menstrual cycle in order to reach a receptive status adequate for embryonic implantation. This period of receptivity is known as the window of implantation (WOI) and occurs between Days 19 and 21 of the menstrual cycle (Navot et al., 1991; Harper, 1992). In any other phase of the menstrual cycle, the endometrium is not receptive to pregnancy (Garrido-Gómez et al., 2013). Successful implantation requires therefore a viable embryo and synchrony between it and the receptive endometrium (Teh et al., 2016). The correct identification and prediction of the period of uterine receptivity is essential to maximize the effectiveness of assisted reproduction treatments.

The study of endometrial receptivity is not new as histological analysis has been traditionally used for endometrial dating (Noyes et al. 1950); however, the accuracy of this method to predict endometrial receptivity has been shown to be limited (Coutifaris et al., 2004; Murray et al., 2004). Some alternative methods to evaluate endometrial receptivity have been developed in the last decade: biochemical markers (Zhang et al., 2012), soluble ligands (Thouas et al., 2015), hormone receptors (Aghajanova et al., 2009), cytokines (Paiva et al., 2011), microRNAs (Sha et al., 2011) or HOX-class homeobox genes (Kwon and Taylor, 2004).

Other studies, focused on the understanding of the molecular mechanisms underlying the histological changes of the endometrium during the menstrual cycle, have identified specific genes responsible for the alterations observed (Talbi et al., 2006). Some other reports have addressed this molecular analysis from a wider perspective, performing a global screening of the transcriptome at different moments of the menstrual cycle (Carson, 2002; Ponnampalam et al., 2004), or under different infertility conditions (Koler et al., 2009; Altmäe et al., 2010), pathologies (Kao et al., 2003) or ovarian stimulation protocols (Horcajadas et al., 2005). Valuable information about the process of endometrial proliferation can be extracted from these studies. However, even though the list of studies is long, the number of molecular diagnostic tools to identify the moment of uterine receptivity is short (Lessey et al., 1995 Dubowy et al., 2003; Díaz-Gimeno et al., 2011).

Therefore, there is a need for more accurate and easy to use diagnostic tools to assess the receptivity status of an endometrium and to maximize the effectiveness of assisted reproduction treatments.

### SUMMARY

In a first aspect, the present invention relates to kit for determining the receptivity status of an endometrium comprising means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1.

### DESCRIPTION

The identification of the optimal time for embryo transfer is essential to maximize the effectiveness of assisted reproduction treatment. The inventors have surprisingly found a highly reliable test for endometrial receptivity, capable of determining if the status of an endometrium is receptive or non-receptive. Moreover, in the case of a non-receptive endometrium, the test developed by the inventors is capable of distinguishing between a pre-receptive and post-receptive endometrium.

The present invention provides a very useful tool for clinical practice routine to help guide embryo transfers to be performed at the best endometrial moment, guaranteeing embryo-endometrial synchrony and thus, allowing for the achievement of better assisted reproduction treatment results. Couples with repeated implantation failure or previously failed in vitro fertilization cycles or couples with recurrent miscarriage can benefit from the detailed analysis of endometrial receptivity and embryo-endometrial synchronization.

The present invention is a new step in the field of personalized medicine in human reproduction in the management of the endometrium in preparation for embryo transfer, with the final goal of achieving better assisted reproduction treatment results, increasing embryo implantation rates and the likelihood of successful pregnancies.

In a preferred embodiment of the present invention, the kit of the first aspect further comprises means for quantifying the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a preferred embodiment of the present invention, the kit of the first aspect comprises means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a preferred embodiment of the present invention, the kit of the first aspect comprises means for quantifying the expression of substantially only the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3. As used herein, the expression "substantially only" means that if any other gene is quantified, then said other gene or genes is or are reference genes, which are only quantified for serving as a reference for the quantification of the expression, as a reference for the sample load.

In a preferred embodiment of the present invention the means for quantifying the genes comprise a pair of primers for each one of the genes.

In a preferred embodiment of the present invention, the kit of the first aspect further comprises a pair of primers for at least one reference gene. Preferably, the reference gene or genes is or are selected from actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I, tyrosine 3-monooxygenase/tryptophan 5-monooxygenase activation protein zeta and combinations thereof. In a preferred embodiment, the kit of the first aspect comprises means for quantifying the expression of all the eight reference genes mentioned above. In another preferred embodiment, the reference genes are cytochrome C1, EMG1 N1-specific pseudouridine methyltransferase and glyceraldehyde-3-phosphate dehydrogenase (CYC1, EMG1, GAPDH).

Therefore, in a preferred embodiment, the kit of the invention consists essentially of means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3, actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I and tyrosine 3-monooxygenase/tryptophan 5-monooxygenase activation protein zeta. As used herein, the expression "consists essentially of" means that no other genes are quantified and that the kit may contain other reagents such as buffers, polymerase enzymes, nucleotides mixes, etc, which are necessary for the analysis of the expression of the above mentioned genes.

In a preferred embodiment of the present invention, the kit of the first aspect further comprises a master mix comprising a DNA-binding dye.

In another preferred embodiment of the present invention, the kit of the first aspect further comprises a specific probe for each one of the genes. Preferably the probe is labeled, preferably dual-labeled. As used herein, the term "labeled" means that the probe is marked by means of a fluorescent reporter dye on the 5' base and a quencher located on the 3' base.

In a preferred embodiment of the present invention, the kit of the first aspect further comprises at least one of the following: a DNA polymerase, a nucleotide mix, a buffer, DNase-free water.

In a second aspect, the present invention relates to a method for determining the receptivity status of an endometrium comprising the quantification of the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, in a sample.

In a preferred embodiment of the second aspect, the method further comprises the quantification of the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a more preferred embodiment of the second aspect of the invention, the expression of the following genes is quantified: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a preferred embodiment of the present invention, the method of the second aspect further comprises quantifying the expression of at least one reference gene in the sample. Preferably, the reference gene or genes is or are selected from actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I, tyrosine 3-monooxygenase/tryptophan 5-monooxygenase activation protein zeta and combinations thereof. In another preferred embodiment, the reference genes are cytochrome C1, EMG1 N1-specific pseudouridine methyltransferase and glyceraldehyde-3-phosphate dehydrogenase (CYC1, EMG1, GAPDH).

In a preferred embodiment of the second aspect of the invention, the quantification is made by qPCR, preferably by RT-qPCR.

In a more preferred embodiment of the second aspect of the invention, the sample is a cDNA sample, preferably a cDNA obtained from an RNA sample from a female subject, preferably a female human. More preferably, the RNA has been obtained from an endometrial biopsy.

In a preferred embodiment of the method of the invention, the results of the gene expression levels can classify an endometrium into: "receptive" or "non receptive". When an endometrium is classified as "receptive", it means that the window of implantation matches the day on which the biopsy was taken. The embryo transfer should be scheduled to be performed on the same day and type of cycle on which the biopsy was taken. If an endometrium is classified as "non-receptive" (either pre-receptive or post-receptive), it means that the WOI is displaced. A new endometrial biopsy on the day estimated needs to be obtained so that the WOI is identified and a personalized embryo transfer is scheduled.

The present invention relates to a method for determining if the status of an endometrium is receptive or non-receptive, and in case it is non-receptive, if it is pre-receptive or post-receptive. The method comprises analyzing in a sample obtained from an endometrium, preferably a human endometrium, the gene expression levels of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

The mRNA of an endometrial biopsy is extracted and purified by methods known in the art. The expression levels of the above mentioned genes are determined and said expression levels are analyzed by a computer software comprising a specific prediction model which classifies and determined the status of the endometrium depending on the gene expression levels. The prediction model is a mathematical system using different algorithms, formulas, to distinguish between a receptive profile, a pre-receptive profile and a post-receptive profile.

### EXAMPLES

### RNA extraction and cDNA preparation

Total RNA was extracted using RNeasy mini kit (Qiagen, London, UK); RNA purity and concentration was confirmed by NanoDrop 2000 Spectophotometer (Thermo Scientific, Waltham, MA, USA) and RNA integrity was assessed using Agilent Bioanalyzer 2100 (Agilent Technologies, Santa Clara, CA, USA) following the manufacturer's instructions. RNAs were reverse-transcribed into cDNA using Fluidigm Reverse Transcription Master Mix (Fluidigm, San Francisco, CA, USA) and either immediately used or stored at -20°C until further downstream processing.

### Gene expression analysis

The pairs of primers targeting the selected and reference genes were designed and are indicated in the sequence listing. The following table includes the primer sequences:

| **GENE** | **PRIMER** | **SEQ ID NO:** | **SEQUENCE** | **Gene Full Name** |
|---|---|---|---|---|
| ANXA4 | forward (F) | 1 | CAGGAGATCAGGACAGCCTA | annexin A4 |
| | reverse (R) | 2 | AGTTGCCACTCAGTTCTGAC | |
| AQP3 | F | 3 | TACCCCTCTGGACACTTGGATA | aquaporin 3 (Gill blood group) |
| | R | 4 | CAACAATGGCCAGCACACA | |
| AREG | F | 5 | GGTGGTGCTGTCGCTCTT | amphiregulin |
| | R | 6 | GCTTCCCAGAGTAGGTGTCATT | |
| ARG2 | F | 7 | AGCTGTGTCAGATGGCTACA | arginase 2 |
| | R | 8 | GGCATGGCCACTAATGGTAC | |
| ATP5B | F | 9 | TGTCGATCTGCTAGCTCCCTA | ATP synthase, H+ transporting, mitochondrial F1 complex, beta polypeptide |
| | R | 10 | TCAGTACAGTCTTGCCAACTCC | |
| CAPN6 | F | 11 | TGGAAAGGTGGTGTGGAAAC | calpain 6 |
| | R | 12 | GTCAGCTGGTGGTTGCTAA | |
| CIR1 | F | 13 | TCAATGCAAGTTCGGTTCCC | corepressor interacting with RBPJ, 1 |
| | R | 14 | CAAACCCACTGTTTCTCATCTCC | |
| CMTM5 | F | 15 | AAGGGCATCCTGCTGGAAA | CKLF-like MARVEL transmembrane domain containing 5 |
| | R | 16 | CAGAGATGGAGGCCGTGAA | |
| CTNNA2 | F | 17 | TGGAAATCCGGACGCTAACA | catenin (cadherin-associated protein), alpha 2 |
| | R | 18 | GCCTTTGTTGCTTGTGTTGAC | |
| CXCL1 | F | 19 | CTTGCCTCAATCCTGCATCC | chemokine (C-X-C motif) ligand 1 (melanoma growth stimulating activity, alpha) |
| | R | 20 | AGCCACCAGTGAGCTTCC | |
| CXCL6 | F | 21 | ACGCTGAGAGTAAACCCCAAA | chemokine (C-X-C motif) ligand 6 |
| | R | 22 | CAAACTTGCTTCCCGTTCTTCA | |
| FOXP3 | F | 23 | TGTGGGGTAGCCATGGAAA | forkhead box P3 |
| | R | 24 | GGGTCGCATGTTGTGGAA | |
| GPX3 | F | 25 | TTTGTCAACGTGGCCAGCTA | glutathione peroxidase 3 (plasma) |
| | R | 26 | AAAGCCCAGAATGACCAGACC | |
| HBA1 | F | 27 | TCTCCTGCCGACAAGACCAA | hemoglobin, alpha 1 |
| | R | 28 | GTGGTGGGGAAGGACAGGA | |
| HBG1 | F | 29 | GCAAGAAGGTGCTGACTTCC | hemoglobin, gamma A |
| | R | 30 | GCTTGTCACAGTGCAGTTCA | |
| HMBS | F | 31 | AGAGTGATTCGCGTGGGTA | hydroxymethylbilane synthase |
| | R | 32 | CTTTCAATGTTGCCACCACAC | |
| HOXB7 | F | 33 | TTCCGGATCTACCCCTGGAT | homeobox B7 |
| | R | 34 | TCTGGTAGCGGGTGTAGGT | |
| IL1R1 | F | 35 | GGGCAAGCAATATCCTATTACCC | interleukin 1 receptor, type I |
| | R | 36 | TAGCTGGGCTCACAATCACA | |
| IL4 | F | 37 | CAGCTGATCCGATTCCTGAAA | interleukin 4 |
| | R | 38 | GTTGGCTTCCTTCACAGGAC | |
| IL5 | F | 39 | ACTCTGAGGATTCCTGTTCCTGTA | interleukin 5 (colony-stimulating factor, eosinophil) |
| | R | 40 | CCAGTGTGCCTATTCCCTGAAA | |
| ITGB1 | F | 41 | GAATGTATACAAGCAGGGCCAAA | integrin, beta 1 (fibronectin receptor, beta polypeptide, antigen CD29 includes MDF2, MSK12) |
| | R | 42 | CGTGCAGAAGTAGGCATTCC | |
| ITGA9 | F | 43 | AAATGCAGCGTGGGATTTCC | integrin, alpha 9 |
| | R | 44 | CCAGACAGGTGGCTTGTATCA | |
| MAOA | F | 45 | ATGGTCTCGGGAAGGTGAC | monoamine oxidase A |
| | R | 46 | GGTGATTTCTACCGCTGGAAC | |
| MAPK1 | F | 47 | TTGGTACAGGGCTCCAGAAA | mitogen-activated protein kinase 1 |
| | R | 48 | TCTGCCAGAATGCAGCCTA | |
| MMP9 | F | 49 | AGTGGCACCACCACAACA | matrix metallopeptidase 9 (gelatinase B, 92kDa gelatinase, 92kDa type IV collagenase) |
| | R | 50 | GCAAAGGCGTCGTCAATCA | |
| MT1F | F | 51 | TCCTCGGCTTGCAATGGA | metallothionein 1F |
| | R | 52 | CTTTGCACTTGCAGGAACCA | |
| MT1H | F | 53 | GCTCCTGCAAGTGCAAAA | metallothionein 1H |
| | R | 54 | GCAGCTGCACTTCTCTGAC | |
| MT1L | F | 55 | CAGCTCCTGCAAGTGCAAA | metallothionein 1L (gene/pseudogene) |
| | R | 56 | GACGCCCCTTTGCAGAC | |
| MT1X | F | 57 | CCTCGAAATGGACCCCAACT | metallothionein 1X |
| | R | 58 | GCACTCTTTGCATTTGCAGGA | |
| NFKBIA | F | 59 | CACCTCCACTCCATCCTGAA | nuclear factor of kappa light polypeptide gene enhancer in B-cells inhibitor, alpha |
| | R | 60 | GGTAGCCATGGATAGAGGCTAA | |
| PGR | F | 61 | AGCCAAGAAGAGTTCCTCTGTA | progesterone receptor |
| | R | 62 | TTGACTTCGTAGCCCTTCCA | |
| PGRMC1 | F | 63 | GGCAAGGTGTTCGATGTGAC | progesterone receptor membrane component 1 |
| | R | 64 | CTCTTCCAGCAAAGACCCCATA | |
| PLA2G4A | F | 65 | AGGCTCCACAATGGAGGAA | phospholipase A2, group IVA (cytosolic, calcium-dependent) |
| | R | 66 | TCATCATCACTGTCCGAGCTA | |
| PTGS1 | F | 67 | GCGGCTCTTTAAGGATGGGAA | prostaglandin-endoperoxide synthase 1 (prostaglandin G/H synthase and cyclooxygenase) |
| | R | 68 | AACACAGGCGCCTCTTCTAC | |
| RHOA | F | 69 | GTGCCCACAGTGTTTGAGAA | ras homolog family member A |
| | R | 70 | TGTGTCCCACAAAGCCAAC | |
| RPL13A | F | 71 | GAGGCCCCTACCACTTCC | ribosomal protein L13a |
| | R | 72 | GCCGTCAAACACCTTGAGAC | |
| SCGB2A2 | F | 73 | CTCTCCCAGCACTGCTAC | secretoglobin, family 2A, member 2 |
| | R | 74 | CTTGTGGATTGATTGTCTTGGAA | |
| SDHA | F | 75 | ACATCGGAACTGCGACTCA | succinate dehydrogenase complex, subunit A, flavoprotein (Fp) |
| | R | 76 | TTCTTGCAACACGCTTCCC | |
| SERPINA1 | F | 77 | CCACGATATCATCACCAAGTTCC | serpin peptidase inhibitor, clade A (alpha-1 antiproteinase, antitrypsin), member 1 |
| | R | 78 | TTGACCCAGGACGCTCTTCA | |
| TFF3 | F | 79 | GGCCTTGCTGTCCTCCA | trefoil factor 3 (intestinal) |
| | R | 80 | CCCGGTTGTTGCACTCC | |
| ACTB | F | 81 | CCAACCGCGAGAAGATGAC | actin, beta |
| | R | 82 | TAGCACAGCCTGGATAGCAA | |
| B2M | F | 83 | TTAGCTGTGCTCGCGCTAC | beta-2-microglobulin |
| | R | 84 | CTCTGCTGGATGACGTGAGTAA | |
| CYC1 | F | 85 | CCATGGCCCCTCCCATCTA | cytochrome c-1 |
| | R | 86 | GGTGCACACATCCTTGGCTA | |
| EMG1 | F | 87 | CAGACCCGAATTCCCAGAAC | EMG1 N1-specific pseudouridine methyltransferase |
| | R | 88 | CTCGAACACTGAGCTTGTGTAA | |
| GAPDH | F | 89 | GAACGGGAAGCTTGTCATCAA | glyceraldehyde-3-phosphate dehydrogenase |
| | R | 90 | A TCGCCCCACTTGATTTTGG | |
| TBP | F | 91 | TGCCCGAAACGCCGAATATA | TATA box binding protein |
| | R | 92 | CGTGGTTCGTGGCTCTCTTA | |
| TOP1 | F | 93 | ACGCTACAGCAGCAGCTAA | topoisomerase (DNA) I |
| | R | 94 | AGCTCGATTGGCACGGTTA | |
| YWHAZ | F | 95 | AGACAGCACGCTAATAATGCA | tyrosine 3-monooxygenase/tryptophan 5-monooxygenase activation protein, zeta |
| | R | 96 | AGCTTCGTCTCCTTGGGTA | |

Specific target amplification (STA) was carried out on cDNA samples using Fluidigm PreAmp Master Mix and DELTAgene assays following the manufacturer's instructions (Fluidigm, San Francisco, CA). RT-qPCR reactions were performed following the Fast Gene Expression Analysis Using Evagreen on the Biomark HD Systema nd 96.96 Dynamic Array™ IFC (Fluidigm, San Francisco, CA). Data was collected with Fluidigm® Real-Time PCR analysis software using linear baseline correction method and global auto Cq threshold method. Data were then exported to Excel as.csv files and Cq values were normalized using the mean of the reference genes selected.

### Study design

Gene expression data from endometrial biopsies obtained at different moments of the menstrual cycle from healthy fertile donors (Group A) and subfertile women (Group B) were analyzed.

### Patient selection and sample collection

Group A consisted of 96 healthy fertile donors (18-34 years), with regular menstrual cycles and normal BMI (25-30). Endometrial biopsies from this group were obtained on two different days of the same natural menstrual cycle: LH + 2, i.e. 2 days after the luteinizing hormone surge and LH + 7, i.e. 7 days after the LH surge. Group B consisted of 120 subfertile patients (30-42 years) undergoing hormone-replacement therapy cycles. Endometrial biopsies from this group of patients were obtained after five full days of progesterone impregnation (P4+5). Endometrial biopsies of approximately 30 mg were obtained from the uterine fundus using a Pipelle catheter (Gynetics, Namont-Achel, Belgium) under sterile conditions. Tissue was then placed in a CryoTube® (Nunc, Roskilde, Denmark) containing 1 ml RNAlater® (Sigma-Aldrich, St Louis, MO, USA) and stored at -20°C until further processing.

### Principal component analysis and discriminant functional analysis

Differential expression of genes from LH + 2 and LH + 7 groups was assessed by comparing ΔCq values (paired t-test (P < 0.05)). Fold change (-ΔΔCq) was calculated to determine upregulated and downregulated genes in the WOI. In order to assess if receptivity status could be established with a reduced number of genes, a principal component analysis (PCA) of the genes showing significant fold change between LH + 2 and LH + 7 was performed. Discriminant functional analysis (DA) was then used to evaluate the ability of the genes with the highest absolute coefficient value from each of the leading principal components to accurately discriminate samples into the following states: proliferative, receptive, pre-receptive and post-receptive. A Split- Sample validation of the DA was performed to assess the reliability and robustness of discriminant findings. Both fertile and infertile patient samples were split into two subsets. One data set (70% of the samples) was used as a training set and the other one as testing set (remaining 30% of the samples). The percentage of correct classifications was calculated to determine the reliability of the DA model. Data analyses were performed by using IBM SPSS Statistics software version 19.0.

### REFERENCES

Aghajanova L et al. Fertil Steril 2009;91:2602-2610.
Altmäe S et al. Mol Hum Reprod 2010;16:178-187.
Carson DD. Mol Hum Reprod 2002;8:871-879.
Coutifaris C et al. Fertil Steril 2004;82:1264-1272.
Dubowy RL et al. Fertil Steril 2003;80:146-156.
Díaz-Gimeno P et al. Fertil Steril 2011;95: 50-60. 60-15.
Garrido-Gómez T et al. Fertil Steril 2013;99:1078-1085.
Giudice LC, Irwin JC. Semin Reprod Endocrinol 1999;17:13-21.
Harper MJK. Baillieres Clin Obstet Gynaecol 1992;6:351-371.
Horcajadas JA et al. Mol Hum Reprod 2005;11:195-205.
Kao LC et al. Endocrinology 2003;144:2870-2881.
Koler M et al. Hum Reprod 2009;24:2541-2548.
Kwon HE, Taylor HS. Ann N Y Acad Sci 2004;1034:1-18.
Lessey BA et al. Fertil Steril 1995;63:535-542.
Murray MJ et al. Fertil Steril 2004;81:1333-1343.
Navot D et al. Fertil Steril 1991;55:114-118.
Noyes RW, Hertig AT, Rock J. Fertil Steril 1950;1:561-564.
Paiva P et al. Hum Reprod 2011;26:1153-1162.
Ponnampalam AP et al. Mol Hum Reprod 2004;10:879-893.
Sha AG et al. Fertil Steril 2011;96:150-155.e5.
Singh M J Endocrinol 2011;210:5-14.
Talbi S et al. 2006;147:1097-1121.
Teh WT et al. J Assist Reprod Genet 2016;33:1419-1430.
Thouas GA et al. Endocr Rev 2015;36:92-130.
Zhang D et al. Reprod Biol Endocrinol 2012;10:106.

## Claims

1. A kit for determining the receptivity status of an endometrium comprising means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1.

2. The kit according to claim 1, further comprising means for quantifying the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

3. The kit according to any one of claims 1 or 2, comprising means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

4. The kit according to any one of claims 1 to 3, wherein the means for quantifying the genes comprise a pair of primers for each one of the genes.

5. The kit according to any one of claims 1 to 4, further comprising a pair of primers for at least one reference gene.

6. The kit according to any one of claims 1 to 5, wherein the reference gene or genes is or are selected from actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I, tyrosine 3-monooxygenase/tryptophan 5-monooxygenase activation protein zeta and combinations thereof.

7. The kit according to any one of claims 1 to 6, further comprising a specific probe for each one of the genes.

8. The kit according to claim 7, wherein the probe is labeled, preferably dual-labeled.

9. The kit according to any one of claims 1 to 8, further comprising at least one of the following: a DNA polymerase, a nucleotide mix, a buffer, DNase-free water.

10. A method for determining the receptivity status of an endometrium comprising the quantification of the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, in a sample.

11. The method according to claim 10, further comprising the quantification of the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

12. The method according to any one of claims 10 or 11, wherein the expression of the following genes is quantified: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

13. The method according to any one of claims 10 to 12, further comprising quantifying the expression of at least one reference gene in the sample.

14. The method according to any one of claims 10 to 13, wherein the quantification is made by qPCR, preferably by RT-qPCR.

15. The method according to any one of claims 10 to 14, wherein the sample is a cDNA sample, preferably a cDNA obtained from an RNA sample from a female subject, preferably a female human.


SEQUENCE LISTING

<110>  Integrated Genetic Lab Services SLU

<120>  Kit and method for determining the receptivity status of an
       endometrium

<130>  EP-1816

<160>  96

<170>  PatentIn version 3.5

<210>  1
<211>  20
<212>  DNA
<213>  Homo sapiens

<400>  1
caggagatca ggacagccta                                                   20


<210>  2
<211>  20
<212>  DNA
<213>  Homo sapiens

<400>  2
agttgccact cagttctgac                                                   20


<210>  3
<211>  22
<212>  DNA
<213>  Homo sapiens

<400>  3
tacccctctg gacacttgga ta                                                22


<210>  4
<211>  19
<212>  DNA
<213>  Homo sapiens

<400>  4
caacaatggc cagcacaca                                                    19


<210>  5
<211>  18
<212>  DNA
<213>  Homo sapiens

<400>  5
ggtggtgctg tcgctctt                                                     18


<210>  6
<211>  22
<212>  DNA
<213>  Homo sapiens

<400>  6
gcttcccaga gtaggtgtca tt                                                22

<210>  7
<211>  20
<212>  DNA
<213>  Homo sapiens

<400>  7
agctgtgtca gatggctaca                                                   20

<210>  8
<211>  20
<212>  DNA
<213>  Homo sapiens

<400>  8
ggcatggcca ctaatggtac                                                   20

<210>  9
<211>  21
<212>  DNA
<213>  Homo sapiens

<400>  9
tgtcgatctg ctagctccct a                                                 21

<210>  10
<211>  22
<212>  DNA
<213>  Homo sapiens

<400>  10
tcagtacagt cttgccaact cc                                                22

<210>  11
<211>  20
<212>  DNA
<213>  Homo sapiens

<400>  11
tggaaaggtg gtgtggaaac                                                   20

<210>  12
<211>  19
<212>  DNA
<213>  Homo sapiens

<400>  12
gtcagctggt ggttgctaa                                                    19

<210>  13
<211>  20
<212>  DNA
<213>  Homo sapiens

<400>  13
tcaatgcaag ttcggttccc                                                   20


<210>  14
<211>  23
<212>  DNA
<213>  Homo sapiens

<400>  14
caaacccact gtttctcatc tcc                                               23


<210>  15
<211>  19
<212>  DNA
<213>  Homo sapiens

<400>  15
aagggcatcc tgctggaaa                                                    19


<210>  16
<211>  19
<212>  DNA
<213>  Homo sapiens

<400>  16
cagagatgga ggccgtgaa                                                    19


<210>  17
<211>  20
<212>  DNA
<213>  Homo sapiens

<400>  17
tggaaatccg gacgctaaca                                                   20


<210>  18
<211>  21
<212>  DNA
<213>  Homo sapiens

<400>  18
gcctttgttg cttgtgttga c                                                 21


<210>  19
<211>  20
<212>  DNA
<213>  Homo sapiens

<400>  19
cttgcctcaa tcctgcatcc                                                   20


<210>  20
<211>  18
<212>  DNA
<213>  Homo sapiens

<400>  20
agccaccagt gagcttcc                                                    18


<210>  21
<211>  21
<212>  DNA
<213>  Homo sapiens

<400>  21
acgctgagag taaaccccaa a                                                21


<210>  22
<211>  22
<212>  DNA
<213>  Homo sapiens

<400>  22
caaacttgct tcccgttctt ca                                               22


<210>  23
<211>  19
<212>  DNA
<213>  Homo sapiens

<400>  23
tgtggggtag ccatggaaa                                                   19


<210>  24
<211>  18
<212>  DNA
<213>  Homo sapiens

<400>  24
gggtcgcatg ttgtggaa                                                    18


<210>  25
<211>  20
<212>  DNA
<213>  Homo sapiens

<400>  25
tttgtcaacg tggccagcta                                                  20


<210>  26
<211>  21
<212>  DNA
<213>  Homo sapiens

<400>  26
aaagcccaga atgaccagac c                                                21


<210>  27
<211>  20
<212>  DNA
<213>  Homo sapiens

<400>  27
tctcctgccg acaagaccaa                                                   20


<210>  28
<211>  19
<212>  DNA
<213>  Homo sapiens

<400>  28
gtggtgggga aggacagga                                                    19


<210>  29
<211>  20
<212>  DNA
<213>  Homo sapiens

<400>  29
gcaagaaggt gctgacttcc                                                   20


<210>  30
<211>  20
<212>  DNA
<213>  Homo sapiens

<400>  30
gcttgtcaca gtgcagttca                                                   20


<210>  31
<211>  19
<212>  DNA
<213>  Homo sapiens

<400>  31
agagtgattc gcgtgggta                                                    19


<210>  32
<211>  21
<212>  DNA
<213>  Homo sapiens

<400>  32
ctttcaatgt tgccaccaca c                                                 21


<210>  33
<211>  20
<212>  DNA
<213>  Homo sapiens

<400>  33
ttccggatct acccctggat                                                   20


<210>  34
<211>  19
<212>  DNA
<213>  Homo sapiens

<400>  34
tctggtagcg ggtgtaggt                                                    19


<210>  35
<211>  23
<212>  DNA
<213>  Homo sapiens

<400>  35
gggcaagcaa tatcctatta ccc                                               23


<210>  36
<211>  20
<212>  DNA
<213>  Homo sapiens

<400>  36
tagctgggct cacaatcaca                                                   20


<210>  37
<211>  21
<212>  DNA
<213>  Homo sapiens

<400>  37
cagctgatcc gattcctgaa a                                                 21


<210>  38
<211>  20
<212>  DNA
<213>  Homo sapiens

<400>  38
gttggcttcc ttcacaggac                                                   20


<210>  39
<211>  24
<212>  DNA
<213>  Homo sapiens

<400>  39
actctgagga ttcctgttcc tgta                                              24


<210>  40
<211>  22
<212>  DNA
<213>  Homo sapiens

<400>  40
ccagtgtgcc tattccctga aa                                                22


<210>  41
<211>  23
<212>  DNA
<213>  Homo sapiens

<400>  41
gaatgtatac aagcagggcc aaa                                              23


<210>  42
<211>  20
<212>  DNA
<213>  Homo sapiens

<400>  42
cgtgcagaag taggcattcc                                                  20


<210>  43
<211>  20
<212>  DNA
<213>  Homo sapiens

<400>  43
aaatgcagcg tgggatttcc                                                  20


<210>  44
<211>  21
<212>  DNA
<213>  Homo sapiens

<400>  44
ccagacaggt ggcttgtatc a                                                21


<210>  45
<211>  19
<212>  DNA
<213>  Homo sapiens

<400>  45
atggtctcgg gaaggtgac                                                   19


<210>  46
<211>  21
<212>  DNA
<213>  Homo sapiens

<400>  46
ggtgatttct accgctggaa c                                                21


<210>  47
<211>  20
<212>  DNA
<213>  Homo sapiens

<400>  47
ttggtacagg gctccagaaa                                                  20


<210>  48
<211>  19
<212>  DNA
<213>  Homo sapiens

<400>  48
tctgccagaa tgcagccta                                                    19


<210>  49
<211>  18
<212>  DNA
<213>  Homo sapiens

<400>  49
agtggcacca ccacaaca                                                     18


<210>  50
<211>  19
<212>  DNA
<213>  Homo sapiens

<400>  50
gcaaaggcgt cgtcaatca                                                    19


<210>  51
<211>  19
<212>  DNA
<213>  Homo sapiens

<400>  51
tcctcggctt gcaatggsa                                                    19


<210>  52
<211>  20
<212>  DNA
<213>  Homo sapiens

<400>  52
ctttgcactt gcaggaacca                                                   20


<210>  53
<211>  18
<212>  DNA
<213>  Homo sapiens

<400>  53
gctcctgcaa gtgcaaaa                                                     18


<210>  54
<211>  19
<212>  DNA
<213>  Homo sapiens

<400>  54
gcagctgcac ttctctgac                                                    19


<210>  55
<211>  19
<212>  DNA
<213>  Homo sapiens

<400>  55
cagctcctgc aagtgcaaa                                                    19


<210>  56
<211>  17
<212>  DNA
<213>  Homo sapiens

<400>  56
gacgcccctt tgcagac                                                      17


<210>  57
<211>  20
<212>  DNA
<213>  Homo sapiens

<400>  57
cctcgaaatg gaccccaact                                                   20


<210>  58
<211>  21
<212>  DNA
<213>  Homo sapiens

<400>  58
gcactctttg catttgcagg a                                                 21


<210>  59
<211>  20
<212>  DNA
<213>  Homo sapiens

<400>  59
cacctccact ccatcctgaa                                                   20


<210>  60
<211>  22
<212>  DNA
<213>  Homo sapiens

<400>  60
ggtagccatg gatagaggct aa                                                22


<210>  61
<211>  22
<212>  DNA
<213>  Homo sapiens

<400>  61
agccaagaag agttcctctg ta                                                22


<210>  62
<211>  20
<212>  DNA
<213>  Homo sapiens

<400>  62
ttgacttcgt agcccttcca                                                    20


<210>  63
<211>  20
<212>  DNA
<213>  Homo sapiens

<400>  63
ggcaaggtgt tcgatgtgac                                                    20


<210>  64
<211>  22
<212>  DNA
<213>  Homo sapiens

<400>  64
ctcttccagc aaagacccca ta                                                 22


<210>  65
<211>  19
<212>  DNA
<213>  Homo sapiens

<400>  65
aggctccaca atggaggaa                                                     19


<210>  66
<211>  21
<212>  DNA
<213>  Homo sapiens

<400>  66
tcatcatcac tgtccgagct a                                                  21


<210>  67
<211>  21
<212>  DNA
<213>  Homo sapiens

<400>  67
gcggctcttt aaggatggga a                                                  21


<210>  68
<211>  20
<212>  DNA
<213>  Homo sapiens

<400>  68
aacacaggcg cctcttctac                                                    20


<210>  69
<211>  20
<212>  DNA
<213>  Homo sapiens

<400>  69
gtgcccacag tgtttgagaa                                                    20


<210>  70
<211>  19
<212>  DNA
<213>  Homo sapiens

<400>  70
tgtgtcccac aaagccaac                                                     19


<210>  71
<211>  18
<212>  DNA
<213>  Homo sapiens

<400>  71
gaggccccta ccacttcc                                                      18


<210>  72
<211>  20
<212>  DNA
<213>  Homo sapiens

<400>  72
gccgtcaaac accttgagac                                                    20


<210>  73
<211>  18
<212>  DNA
<213>  Homo sapiens

<400>  73
ctctcccagc actgctac                                                      18


<210>  74
<211>  23
<212>  DNA
<213>  Homo sapiens

<400>  74
cttgtggatt gattgtcttg gaa                                                23


<210>  75
<211>  19
<212>  DNA
<213>  Homo sapiens

<400>  75
acatcggaac tgcgactca                                                     19


<210>  76
<211>  19
<212>  DNA
<213>  Homo sapiens

<400>  76
ttcttgcaac acgcttccc                                                    19


<210>  77
<211>  23
<212>  DNA
<213>  Homo sapiens

<400>  77
ccacgatatc atcaccaagt tcc                                               23


<210>  78
<211>  20
<212>  DNA
<213>  Homo sapiens

<400>  78
ttgacccagg acgctcttca                                                   20


<210>  79
<211>  17
<212>  DNA
<213>  Homo sapiens

<400>  79
ggccttgctg tcctcca                                                      17


<210>  80
<211>  17
<212>  DNA
<213>  Homo sapiens

<400>  80
cccggttgtt gcactcc                                                      17


<210>  81
<211>  19
<212>  DNA
<213>  Homo sapiens

<400>  81
ccaaccgcga gaagatgac                                                    19


<210>  82
<211>  20
<212>  DNA
<213>  Homo sapiens

<400>  82
tagcacagcc tggatagcaa                                                   20


<210>  83
<211>  19
<212>  DNA
<213>  Homo sapiens

<400>  83
ttagctgtgc tcgcgctac                                                    19


<210>  84
<211>  22
<212>  DNA
<213>  Homo sapiens

<400>  84
ctctgctgga tgacgtgagt aa                                                22


<210>  85
<211>  19
<212>  DNA
<213>  Homo sapiens

<400>  85
ccatggcccc tcccatcta                                                    19


<210>  86
<211>  20
<212>  DNA
<213>  Homo sapiens

<400>  86
ggtgcacaca tccttggcta                                                   20


<210>  87
<211>  20
<212>  DNA
<213>  Homo sapiens

<400>  87
cagacccgaa ttcccagaac                                                   20


<210>  88
<211>  22
<212>  DNA
<213>  Homo sapiens

<400>  88
ctcgaacact gagcttgtgt aa                                                22


<210>  89
<211>  21
<212>  DNA
<213>  Homo sapiens

<400>  89
gaacgggaag cttgtcatca a                                                 21


<210>  90
<211>  20
<212>  DNA
<213>  Homo sapiens

<400>  90
atcgccccac ttgattttgg                                                   20


<210>  91
<211>  20
<212>  DNA
<213>  Homo sapiens

<400>  91
tgcccgaaac gccgaatata                                                   20


<210>  92
<211>  20
<212>  DNA
<213>  Homo sapiens

<400>  92
cgtggttcgt ggctctctta                                                   20


<210>  93
<211>  19
<212>  DNA
<213>  Homo sapiens

<400>  93
acgctacagc agcagctaa                                                    19


<210>  94
<211>  19
<212>  DNA
<213>  Homo sapiens

<400>  94
agctcgattg gcacggtta                                                    19


<210>  95
<211>  21
<212>  DNA
<213>  Homo sapiens

<400>  95
agacagcacg ctaataatgc a                                                 21


<210>  96
<211>  19
<212>  DNA
<213>  Homo sapiens

<400>  96
agcttcgtct ccttgggta                                                    19